(19) **Europäisches Patentamt**

**European Patent Office**

**Office européen des brevets**

(11) Veröffentlichungsnummer: **0 150 666**
**B1**

(12) # EUROPÄISCHE PATENTSCHRIFT

(45) Veröffentlichungstag der Patentschrift:
**12.10.88**

(51) Int. Cl.⁴: **A 61 M 25/00**, A 61 M 1/00

(21) Anmeldenummer: **84810451.9**

(22) Anmeldetag: **14.09.84**

(54) Verschluss an einem Harnkatheter.

(30) Priorität: **27.12.83 CH 6933/83**

(43) Veröffentlichungstag der Anmeldung:
**07.08.85 Patentblatt 85/32**

(45) Bekanntmachung des Hinweises auf die Patenterteilung:
**12.10.88 Patentblatt 88/41**

(84) Benannte Vertragsstaaten:
**AT BE CH DE FR GB IT LI LU NL SE**

(56) Entgegenhaltungen:
**EP-A-0 088 871**
**DE-A-2 550 829**
**DE-A-2 953 646**
**DE-A-3 143 643**
**US-A-2 733 713**
**US-A-3 335 727**
**US-A-3 915 167**
**US-A-3 984 081**

(73) Patentinhaber: **Pfister- Lehmann, Alfred, Hauptstrasse, CH- 3706 Leissigen (CH)**

(72) Erfinder: **Pfister- Lehmann, Alfred, Hauptstrasse, CH- 3706 Leissigen (CH)**

(74) Vertreter: **Seehof, Michel, c/o AMMANN PATENTANWAELTE AG BERN Schwarztorstrasse 31, CH- 3001 Bern (CH)**

EP 0 150 666 B1

LIBER, STOCKHOLM 1988

## Beschreibung

Die vorliegende Erfindung betrifft einen Verschluss an einem Harnkatheter, gemäss Oberbegriff des Anspruchs 1.

Ein derartiger Verschluss, allerdings nicht ausdrücklich für Harnkatheter bestimmt, ist aus der DE-A-2 550 829 bekannt. Das Gehäuse ist hierbei lose auf den durchgehend den gleichen Querschnitt aufweisenden Schlauch aufgesetzt und somit nicht ohne weiteres in bestimmter Lage gesichert, besonders wenn der Schlauch nicht zwischen der Druckrolle und dem Gehäuse geklemmt ist.

Ein weiterer bekannter Verschluss für einen Hankatheter ist aus der EP-A-0 088 871 bekannt, der zwei gegenseitig in Durchflussrichtung verschiebbare Gehäuseteile aufweist, zwischen welchen im geschlossenen Zustand ein Quetschschlauch unter Federdruck eingeklemmt ist. Aufbau und Herstellung dieses Verschlusses sind relativ aufwendig, und es ist nicht möglich, den Verschluss in offenem Zustand zu blockieren, um beispielsweise Spülungen vorzunehmen.

Ziel vorliegender Erfindung ist es, einen in Aufbau und Bedienung einfachen und doch vielseitig verwendbaren und sicheren Katheterverschluss zu schaffen. Dieses Ziel wird gemäss dem Kennzeichen des Anspruchs 1 erreicht. Auf dem einteiligen Schlauch ist das Gehäuse zwischen den genannten Schultern sicher gehalten. Die Druckrolle ist einfach bedienbar und kann in eine stabile Offenstellung gebracht werden. Die Herstellung kann besonders einfach erfolgen, indem vorbereitete Gehäusehälften auf den Schlauch aufgesetzt und verklebt oder verschweisst werden.

Die Erfindung wird nun anhand eines in der Zeichnung dargestellten Ausführungsbeispiels näher erläutert.

Figur 1    zeigt einen Längsschnitt durch das Ausführungsbeispiel und
Figur 2    zeigt einen Schnitt nach Linie II-II in Fig. 1.

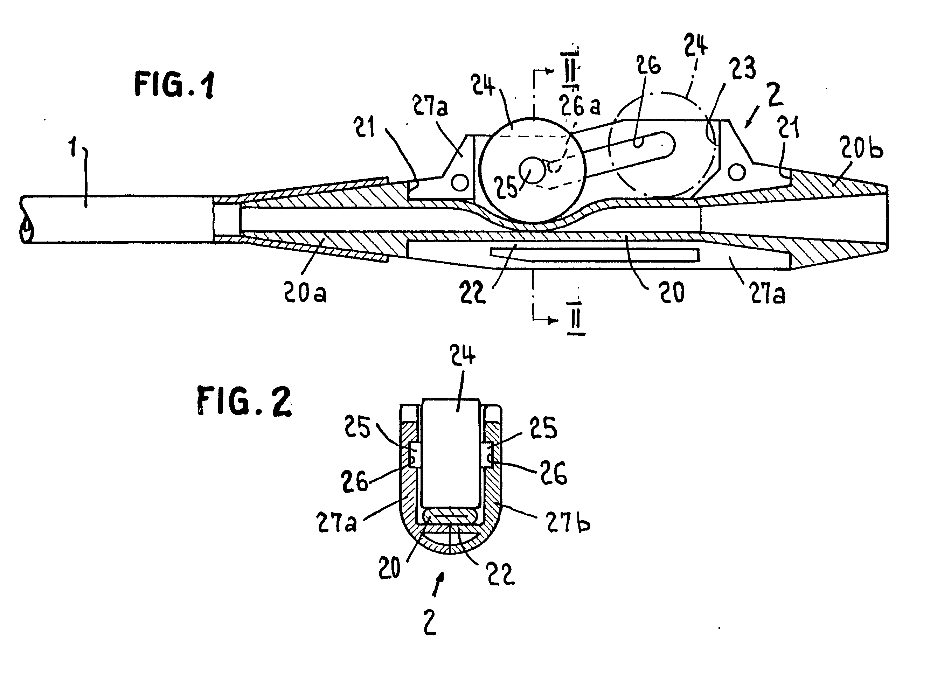

Das dargestellte Ausführungsbeispiel zeichnet sich durch ganz besondere Einfachheit in Aufbau und Bedienung aus. Ein Ventilschlauch 20, z. B. aus Silikonkautschuk, weist an beiden Enden einen Konus 20a bzw. 20b auf, von welchen der eine zum Anschluss an einen Katheter 1, der andere zum eventuellen Anschluss eines Harnauffangbeutels dient. Die beiden konischen Enden weisen innen eine Schulter 21 auf, und zwischen den beiden Schultern weist der Schlauch eine Wandstärke auf, die ein elastisches Zusammendrücken desselben erlaubt. Zwischen den Schultern 21 ist auch ein Gehäuse 2 auf den Schlauch aufgesetzt, das aus zwei symmetrischen Kunststoffteilen 27a, 27b besteht, die in der Symmetrieebene verklebt oder verschweisst sind. Die beiden Gehäusehälften werden bei der Herstellung über den Schlauch gegeneinandergeschoben und verklebt oder verschweisst, wobei die Schultern 21 die Lage des Gehäuses auf dem Schlauch bestimmen. Das Gehäuse weist einen Boden 22 auf, auf welchem sich der Schlauch abstützen kann. Gegenüber dem Boden 22 weist das Gehäuse eine schlitzartige Öffnung 23 auf, in welcher sich eine Druckrolle 24 befindet, die mit zwei seitlichen Zapfen 25 in je eine Nut 26 an jeder Seitenwand der Öffnung 23 greift. Wie Fig. 1 zeigt, ist die Nut 26 in Richtung vom Katheter 1 weg nach aussen geneigt, weist aber an dem dem Katheteranschluss zugewandten Ende auch ein kurzes, nach aussen geneigtes Teilstück 26a auf.

Beim dargestellten Zustand befindet sich die Druckrolle 24 am inneren Ende der Nut 26 in deren nach aussen geneigtem Teilstück oder Ende 26a. Der Schlauch 20 ist zwischen der Druckrolle 24 und dem Boden 22 ganz zusammengedrückt und das Ventil somit geschlossen. Die Druckrolle 24 ist dabei in dieser Schließstellung so sicher gehalten, dass sie auch unter der Wirkung zufälliger Reibung an der Kleidung das nach aussen geneigte Ende 26a der Nut nicht verlassen wird, da ihre Zapfen 25 unter dem elastischen Druck des zusammengedrückten Schlauches nach aussen gegen die Enden der Nuten 26 gedrückt werden. Zum Lösen von Harn kann das Ventil mit einer Hand erfasst und die Druckrolle 24 nach vorne, also sinnfällig in Ausflussrichtung vom Katheter weg, in die in Fig. 1 strichpunktiert dargestellte Lage geschoben werden. Der Schlauch 20 wird dadurch frei und kann sich unter seiner Eigenelastizität ausdehnen und den Harn durchtreten lassen. Ebenso leicht kann sie nachträglich wieder in ihre Schließstellung zurückverbracht werden.

Der dargestellte Verschluss ist nicht nur einfach in der Herstellung, im Aufbau und in der Bedienung, sondern er kann auch in kleinen Abmessungen ausgeführt werden, so dass er problemlos in der Kleidung versorgt werden kann. Bei bettlägerigen Personen kann am vorderen Konus 20b, der etwas grösseren Durchmesser aufweist als der hintere Konus 20a, ein Harnauffangbeutel angeschlossen werden. Das Ventil bleibt übrigens dank der Eigenelastizität des Schlauches 20 auch stabil in seiner Offenstellung, so dass bei dieser Ventilstellung Spülungen vorgenommen werden können. Der Schlauch übernimmt daher trotz seiner einfachen Form mehrfache Funktionen mit seiner Elastizität, seinen beiden konischen Enden oder Stutzen und seinen Schultern 21, die das Gehäuse auf dem Schlauch sichern.

## Patentansprüche

1. Verschluss für einen Harnkatheter, mit einem elastisch verformbaren Schlauch (20), auf welchen ein Gehäuse (2) mit einer verschiebbaren Druckrolle (24) aufgesetzt ist, wobei die Druckrolle längs einer Führung (26) aus einer unwirksamen Endlage, in welcher sie den Schlauch freigibt, in eine wirksame Endlage verschiebbar ist, in welcher sie den Schlauch zusammendrückt und abschliesst, dadurch gekennzeichnet, dass der Schlauch verdickte Endteile (20a, 20b) aufweist, die jeweils über eine Schulter (21) in einen dünnwandigeren Mittelteil übergehen, wobei das Gehäuse zwischen den Schultern (21) gehalten und positioniert ist.

2. Verschluss nach Anspruch 1, dadurch gekennzeichnet, dass die Führung (26) in einem spitzen Winkel zur Längsmittelachse des Schlauches (20) verläuft und an ihrem näher am Schlauch liegenden Ende ein vom Schlauch weg geneigtes Endteil (26a) aufweist, in welchem die Druckrolle (24) unter dem elastischen Druck des zusammengedrückten Schlauches eingerastet ist.

3. Verschluss nach Anspruch 1 oder 2, dadurch gekennzeichnet, dass die Endteile des Schlauches (20) konische Aussenflächen (20a, 20b) zum Anschluss an den Katheter bzw. an einen Auffangbeutel oder eine Spülleitung aufweisen.

4. Verschluss nach einem der Ansprüche 1 - 3, dadurch gekennzeichnet, dass das Ventilgehäuse aus zwei symmetrisch zur Längsachse des Gehäuses verlaufenden Kunststoffteilen (27a, 27b) besteht, die miteinander verklebt oder verschweisst sind.

## Claims

1. A closure at an ureteral catheter, with an elastically deformable tube (20) on which is arranged a housing (2) with a pressure roller (24) movable along a guide (26) from an inactive end position in which it releases the tube to an active end position in which it compresses and closes the tube, characterized in that the tube comprises thickened end parts (20a, 20b) each connected by a shoulder (21) to a thin-walled center part, the housing being held and positioned between the shoulders (21).

2. A closure according to claim 1, characterized in that the guide (26) extends at an acute angle relatively to the central longitudinal axis of the tube (20) and in that it comprises at its extremity closest to the tube an inclined end part (26a) running away from the tube in which the pressure roller (24) is locked by the elastic pressure of the compressed tube.

3. A closure according to claim 1 or 2, characterized in that the end parts of the tube (20) comprise conical external faces (20a, 20b) for a connection to a catheter, resp. to a collecting bag or to a rinsing conduct.

4. A closure according to one of the claims 1 - 3, characterized in that the valve housing consists of two plastic parts (27a, 27b) glued or bonded together extending symmetrically to the longitudinal axis of the housing.

## Revendications

1. Fermeture pour une sonde urétérale, avec un tuyau élastiquement déformable (20) sur lequel est disposé un boîtier (2) avec un rouleau presseur (24) déplaçable le long d'un guide (26) d'une position d'extrémité inactive dans laquelle il libère le tuyau, à une position d'extrémité active dans laquelle il comprime et ferme le tuyau, caractérisée en ce que le tuyau comprend des parties d'extrémité plus épaisses (20a, 20b) reliées chacune par un épaulement 21) à une partie centrale à paroi plus mince, le boîtier étant tenu et positionné entre les épaulements (21).

2. Fermeture selon la revendication 1, caractérisée en ce que le guide (26) s'étend avec un angle aigu relativement à l'axe longitudinal central du tuyau (20) et qu'il comprend à son extrémité la plus rapprochée du tuyau une partie d'extrémité (26a) inclinée s'éloignant du tuyau dans laquelle le rouleau presseur (24) est verrouillé par la pression élastique du tuyau comprimé.

3. Fermeture selon la revendication 1 ou 2, caractérisée en ce que les parties d'extrémité du tuyau (20) ont des faces extérieures coniques (20a, 20b) pour une liaison à un cathéter, resp. à un sachet collecteur ou à une conduite de rinçage.

4. Fermeture selon l'une des revendicatins 1 - 3, caractérisée en ce que le boîtier de soupape consiste en deux parties en plastic (27a, 27b) collées ou soudées ensemble s'étendant symétriquement à l'axe longitudinal du boîtier.

# FIG.1

# FIG.2

0 150 666